# FASCICULE DE BREVET EUROPEEN

(11) **EP 1 201 645 B1**
(45) Date de publication et mention de la délivrance du brevet: **22.09.2004**
(21) Numéro de dépôt: 01402724.7
(22) Date de dépôt: 19.10.2001
(51) Int. Cl.: C07C 219/08, C07C 211/63, C08F 20/34

(54) **Acrylates comportant plusieurs groupes amino quaternaires dans la partie alcool, leur procédé de fabrication et les (co)polymères obtenus à partir de ces monomères**
Acrylate mit mehreren quatenären Amino-Gruppen im Alkohol-Teil, Verfahren zu deren Herstellung, und (Co)Polymere hergestellt aus diesen Monomeren
Acrylates having several quaternary amino groups in the alcohol moiety, process for their preparation and (co)polymers obtained from these monomers

(30) Priorité: 30.10.2000 FR 0013916
(43) Date de publication de la demande: 02.05.2002
(73) Titulaire: Atofina, 92800 Puteaux (FR)
(72) Inventeur: Riondel, Alain, 57600 Forbach (FR); Chaplinski, Vladimir, 60100 Creil (FR)
(74) Mandataire: Rieux, Michel

(56) Documents cités:
- EP-A- 0 839 767
- FR-A- 1 529 000
- US-A- 4 009 201

## Description

La présente invention porte sur de nouveaux monomères acrylates comportant plusieurs groupes amino quaternaires dans la partie alcool, sur leur procédé de fabrication, et sur les nouveaux (co)polymères obtenus à partir de ces nouveaux monomères.

Les composé du type de ceux de formule : 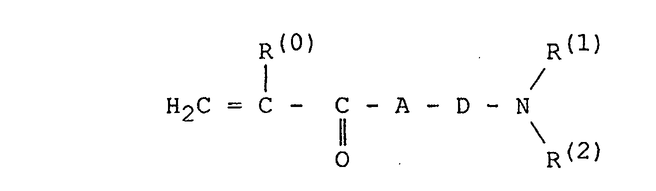 dans laquelle :
- R⁽⁰⁾ représente H ou CH₃ ;
- A représente -O- ou -NH- ;
- D représente une chaîne alkylène linéaire ou ramifiée en C₁-C₆ ;
- R⁽¹⁾ et R⁽²⁾, identiques ou différents, représentent chacun indépendamment H ou alkyle en C₁₋₅ ;
sont bien connus dans la littérature.

Des composés importants de cette famille sont l'acrylate de N,N-diméthylamino éthyle (ADAME) et le méthacrylate de N,N-diméthylamino éthyle (MADAME) : 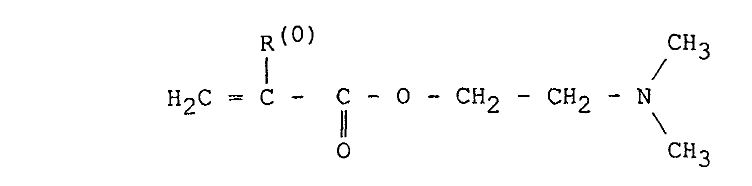 avec R⁽⁰⁾ = H ou CH₃.

Une très nombreuse littérature-brevets décrit la fabrication de solutions aqueuses de sels d'ammonium quaternaire, à partir de l'ADAME et du MADAME, ces sels, pour les plus représentatifs d'entre eux, pouvant être représentés par la formule : 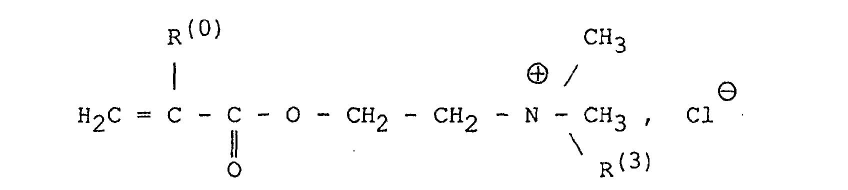 avec R⁽⁰⁾ = H ou CH₃ et R⁽³⁾ = CH₃ ou benzyle,
ces sels pouvant être désignés par l'abréviation (M)ADAMQUAT MC ou (M)ADAMQUAT BZ suivant que R⁽³⁾ représente CH₃ ou benzyle.

Cette réaction est une quaternisation, en présence d'eau, du composé de départ avec un agent quaternisant R⁽³⁾ - Cl.

Les solutions aqueuses de sels quaternaires ainsi obtenues servent notamment à préparer des polymères destinés à servir de floculants cationiques dans le traitement des eaux.

Par le brevet CZ-A-250 962, on connaît les composés de formule : 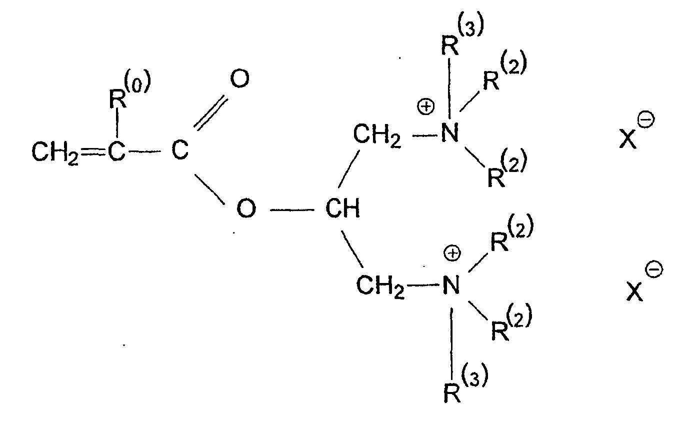 dans laquelle :
- R⁽⁰⁾ représente H ou -CH₃ ;
- R⁽²⁾ représente -CH₃, -C₂H₅, -C₃H₇ ou -C₄H₉ ;
- R⁽³⁾ représente -CH₃, -C₂H₅, -C₃H₇, -C₄H₉, C₆H₅ ou -CH₂C₆H₅ ; et
- X^{⊖} représente Cl^{⊖} ou Br^{⊖}.

On connaît également, par FR 2804110 (la demande de brevet français n° 00/00834 du 24 janvier 2000), les composés de formule : 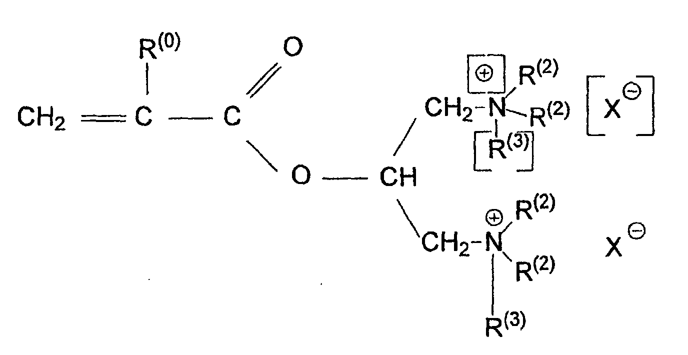 dans laquelle :
- R⁽⁰⁾ représente H ou -CH₃ ;
- R⁽²⁾ représente -CH₃ ; -C₂H₅ ; -C₃H₇ ou -C₄H₉ ; et
le composé étant facultativement quaternisé sur l'un des azotes ; lorsque le composé est quaternisé sur un seul azote, R⁽³⁾ et X^{⊖} ont les significations suivantes: R⁽³⁾ représente -CH₃ ou -CH₂C₆H₅ ; et X^{⊖} représente Cl^{⊖} ou CH₃OSO₃^{⊖} ; ou R⁽³⁾ représente un groupement alkyle en C₁-C₁₂ ; et X^{⊖} représente Br^{⊖} ou I^{⊖} ; lorsque le composé est quaternisé sur les deux azotes, les deux X^{⊖} peuvent être identiques ou différents et les deux R⁽³⁾ peuvent être identiques, auquel cas R⁽³⁾ représente un groupement alkyle en C₅-C₁₂ ; et X^{⊖} représente CH₃OSO₃^{⊖}, Br^{⊖} ou I^{⊖} ; ou différents, auquel cas l'un des R⁽³⁾ représente -CH₃ ou -CH₂C₆H₅ ; et X^{⊖} représente Cl^{⊖} ou CH₃OSO₃^{⊖} ; et l'autre représente un groupe alkyle en C₅-C₁₂ ; et X^{⊖} représente Br^{⊖} ou I^{⊖}.

Tous ces monomères connus permettent d'accéder à des floculants utiles, mais dont la cationicité est jugée encore insuffisante pour certaines applications.

Par le brevet US 4 009 201, on connaît des monomères acrylates comportant deux ou plusieurs groupes amino quaternaires.

La Société déposante a maintenant découvert de nouveaux monomères qui répondent à cette attente. Ces monomères font donc l'objet de la présente invention, ainsi que leur procédé de fabrication et les homo- ou copolymères comportant des motifs dérivés de ces nouveaux monomères.

La présente invention a donc d'abord pour objet un composé de formule (Ia) ou (Ib) : 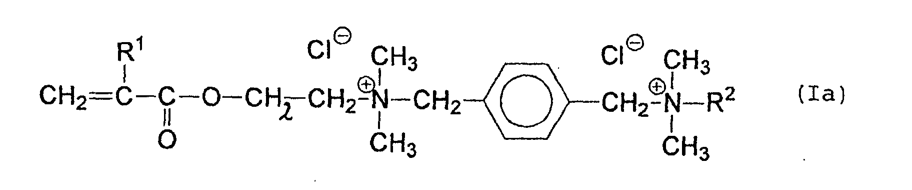 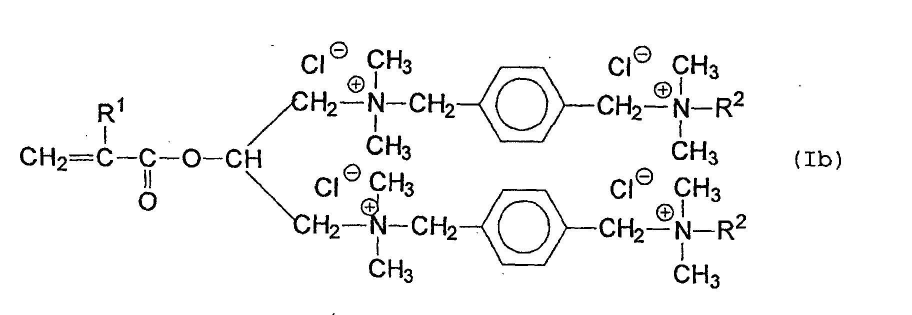 dans lesquelles :
- R¹ représente hydrogène ou méthyle ; et
- R² représente éthyle ou isopropyle.

Les composés de l'invention peuvent avantageusement se présenter en solution aqueuse, ayant une concentration en composé (Ia) ou (Ib) qui est notamment de 60 à 80% en poids.

La présente invention a également pour objet un procédé de fabrication du composé de formule (Ia) ou (Ib), tel que défini ci-dessus, caractérisé par le fait que :
- dans une première étape, on fait réagir le dichlorure de p-xylylène : 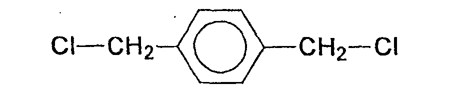 avec un composé de formule (II) : 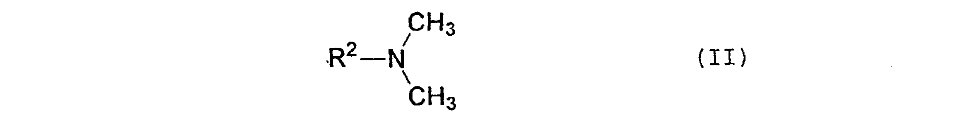 dans laquelle R² est tel que défini ci-dessus, pour obtenir un composé de formule (III) : 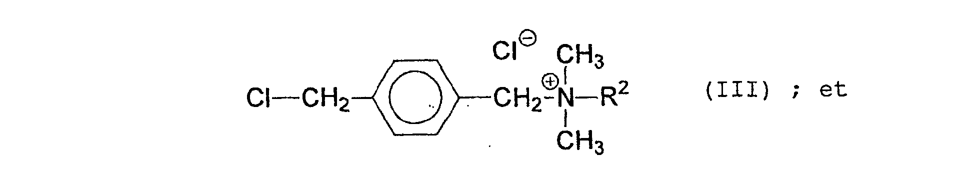
- dans une seconde étape, on fait réagir le composé de formule (III) ainsi obtenu avec un composé de formule (IVa) ou (IVb) : 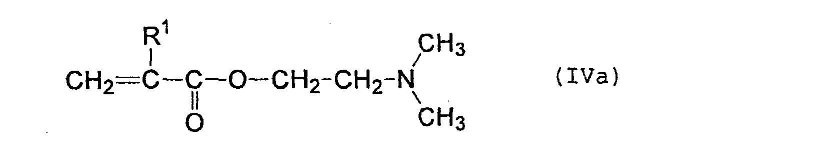 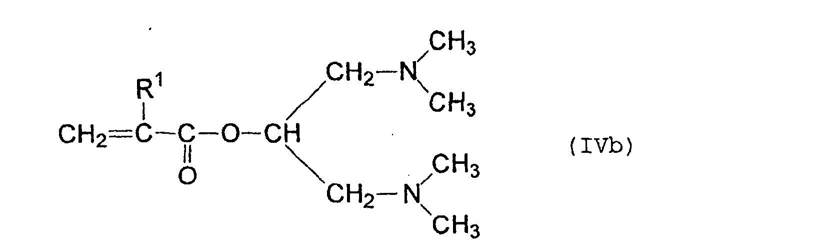
dans lesquelles R¹ est tel que défini ci-dessus, conduisant à une solution aqueuse du composé respectivement (Ia) ou (Ib), dont on élimine l'eau le cas échéant.

À la première étape, on conduit généralement la réaction en milieu solvant, tel que le tétrahydrofurane, avec un rapport molaire du dichlorure de p-xylylène au composé (II) de 0,8 à 1,2, de préférence 0,9 à 1,1, à une température de 5 à 60°C, de préférence de 15 à 40°C, pendant un laps de temps allant de 8 à 144 heures, de préférence de 10 à 96 heures.

Le produit est obtenu sous forme de cristaux incolores.

La réaction de la deuxième étape est conduite avec un rapport molaire du composé (III) au composé (IVa) ou (IVb) compris entre 0,95 et 2,1 selon le cas, à une température de 30 à 70°C, de préférence de 40 à 55°C, pendant un laps de temps de 0,5 à 8 heures, de préférence de 1 à 6 heures.

Par ailleurs, on conduit généralement la seconde étape en présence d'au moins un stabilisant choisi notamment parmi l'éther méthylique de l'hydroquinone, le 3,5-ditert.-butyl-4-hydroxytoluène et l'hydroquinone, et les mélanges de ces stabilisants, à raison de 100 à 2000 ppm par rapport à la charge.

La présente invention a également pour objet des homopolymères ou copolymères comportant des motifs d'au moins un monomère de formule (Ia) ou (Ib), tel que défini ci-dessus.

Les copolymères à base des monomères (Ia) et (Ib) peuvent être des polymères hydrosolubles ou hydrophobes ayant une présentation sous forme de dispersion aqueuse, latex, solution aqueuse, émulsion inverse ou de poudre. Ils sont préparés par copolymérisation radicalaire selon divers procédés de synthèse tels que les procédés de polymérisation en dispersion, solution, émulsion directe, émulsion inverse, suspension inverse et eau dans eau.

La présente invention porte également sur les composés de formule (III) tels que définis ci-dessus à titre de composés intermédiaires dans la préparation des composés (I).

Les Exemples suivants illustrent la présente invention sans toutefois en limiter la portée. Les pourcentages sont en poids sauf indication contraire. Dans ces Exemples, les abréviations suivantes on été utilisées :
- THF : tétrahydrofurane
- ADAME : acrylate de N,N-diméthylaminoéthyle
- S-ADAME : acrylate de 2-(diméthylamino)-1-(diméthylaminométhyl)éthyle
- EMHQ : éther méthylique de l'hydroquinone

### EXEMPLE 1 : Synthèse du chlorure de 4-(chlorométhyl)-N-éthyl-N,N-diméthylbenzèneméthanaminium

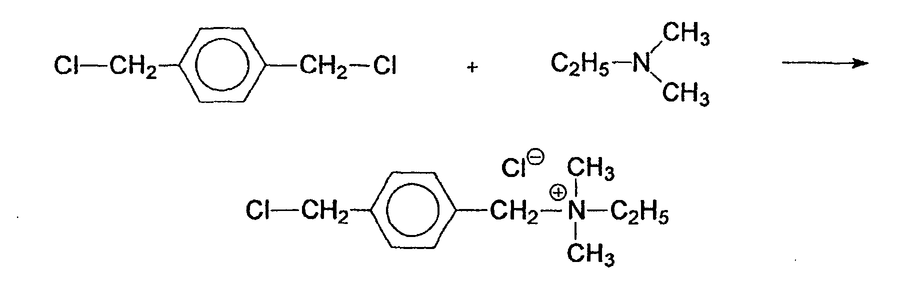

Dans un ballon tricol de 250 ml équipé d'un thermomètre et d'une agitation mécanique, on a placé 8,75 g (50,0 mmoles) de dichlorure de p-xylylène et 100 ml de THF. Le mélange a été agité jusqu'à ce que tous les solides aient été dissous (sa température est tombée à 15°C à ce moment) et 5,64 ml (52,5 mmoles) de N-éthyldiméthylamine ont été ajoutés aussitôt.

Le ballon a ensuite été fermé à l'aide d'un bouchon en verre, et son contenu a été agité pendant 4 jours à 35°C, des échantillons de la solution réactionnelle filtrée étant analysés au bout d'1 jour et de 4 jours. Le dernier échantillon a présenté une conversion de 92% de la matière de départ. Le précipité a été recueilli par filtration et séché sous vide pendant 30 minutes à la température ambiante pour donner 10,56 g du produit attendu sous la forme de cristaux incolores (rendement de 85%).
RMN ¹H (CDCl₃) : δ 7,8-7,3 (m, 4H), 5,09 (s, 2H), 4,56 (s, 2H), 3,66 (q, 2H), 3,25 (s, 6H), 1,43 (t, 3H).
RMN ¹³C (CDCl₃) : δ 140,2(s), 134,0(d), 129,4(d), 128(s), 66,6(t), 59,5(t), 49,1(q), 45,6(t), 8,9(q).

### EXEMPLE 2 : Synthèse du chlorure de 4-(chlorométhyl)-N-i-propyl-N,N-diméthylbenzèneméthanaminium

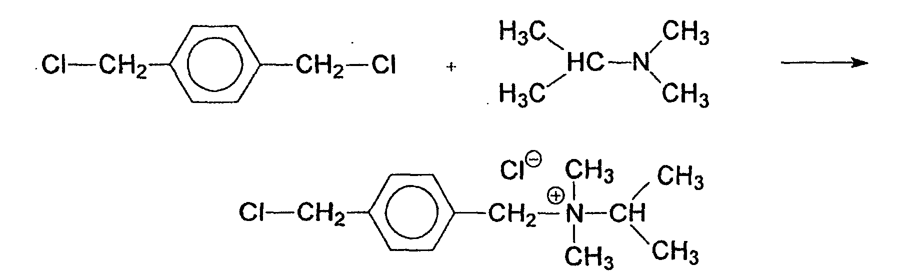

Dans un ballon tricol de 250 ml équipé d'un thermomètre et d'une agitation mécanique, on a placé 8,75 g (50,0 mmoles) de dichlorure de p-xylylène et 100 ml de THF. Le mélange a été agité jusqu'à ce que tous les solides aient été dissous (sa température est tombée à 15°C à ce moment) et 6,39 ml (52,5 mmoles) de N-isopropyldiméthylamine ont été ajoutés aussitôt.

Le ballon a ensuite été fermé à l'aide d'un bouchon en verre, et son contenu a été agité pendant 4 jours à 45°C, des échantillons de la solution réactionnelle filtrée étant analysés au bout d'1 jour, de 2 jours et à la fin. Le dernier échantillon a présenté une conversion de 87% de la matière de départ. Le précipité a été recueilli par filtration et séché pendant 3 heures à la température ambiante et 1,99 x 10³ Pa (15 mm Hg) pour donner 10,35 g du produit attendu sous la forme de cristaux incolores (rendement de 79%).
RMN ¹³C (D₂O) : δ 140,0(s), 133,6(d), 129,7(d), 127,8(s), 66,6(d), 64,5(t), 47,0(q), 45,6(t), 45,8(t), 16,4(q).

### EXEMPLE 3 : Synthèse du dichlorure de N-acryloyloxyéthyl-N'-éthyl-N,N,N',N'-tétraméthylbenzène-1,4-diméthanaminium

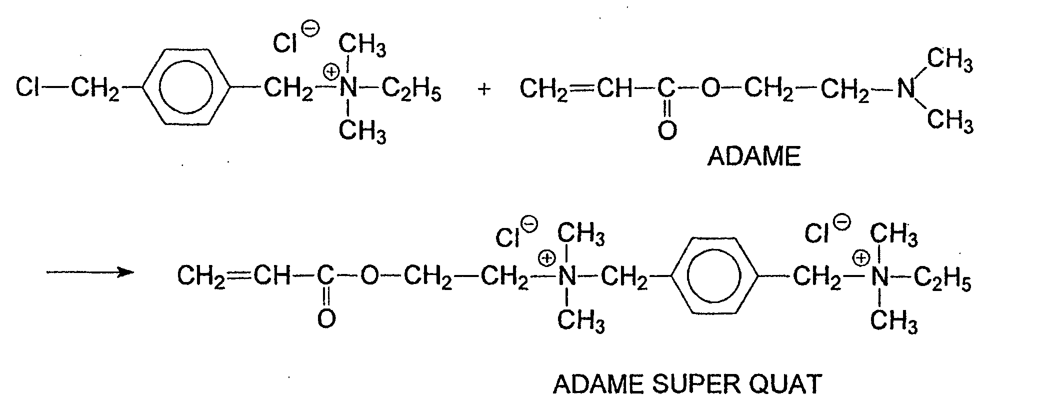

Dans un flacon en verre à large embouchure de 25 ml, équipé d'un capuchon et d'un barreau d'agitation magnétique, on a placé 8,00 g (32,3 mmoles) du composé obtenu à l'Exemple 1, 4,61 g (32,3 mmoles) d'ADAME, 4,20 g d'eau et 16,8 mg (1000 ppm) d'EMHQ.

Le mélange a ensuite été agité sans chauffage ni refroidissement. La température interne s'est tout d'abord élevée jusqu'à 50°C en raison de l'effet exothermique, puis elle a commencé à chuter. Au bout de 3 heures d'agitation à la température ambiante, un mélange homogène très visqueux s'est formé et l'analyse de chlorure ionique a montré que la réaction était terminée (100% de conversion). On a obtenu un rendement quantitatif du produit attendu sous la forme d'une solution à 75% dans l'eau. Sa structure a été confirmée par analyse RMN.
RMN ¹³C (D₂O) : δ 165,0(s), 134,2(d), 134,1(d), 133,8(t), 130,4(s), 129,7(s), 127,4(d), 68,7(t), 66,9(t), 63,3(t), 60,6(t), 58,7(t), 50,9(q), 49,6(q), 8,2(q).

### EXEMPLE 4 : Synthèse du dichlorure de N-acryloyloxyéthyl-N'-i-propyl-N,N,N',N'-tétraméthylbenzène-1,4-diméthanaminium

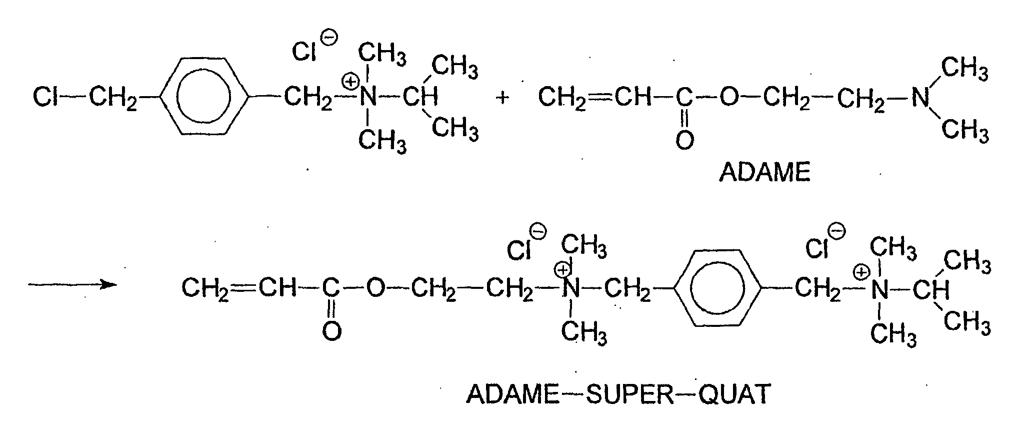

Dans un flacon en verre à large embouchure de 25 ml, équipé d'un capuchon et d'un barreau d'agitation magnétique, on a placé 4,90 g (18,7 mmoles) du composé obtenu à l'Exemple 2, 2,67 g (18,7 mmoles) d'ADAME, 2,52 g d'eau et 10,1 mg (1000 ppm) d'EMHQ.

Le mélange a ensuite été agité sans chauffage ni refroidissement. La température interne s'est tout d'abord élevée jusqu'à 50°C en raison de l'effet exothermique, puis elle a commencé à chuter. Au bout de 1 heure d'agitation à la température ambiante, un mélange homogène très visqueux s'est formé et l'analyse de chlorure ionique a montré que la réaction était terminée (100% de conversion). On a obtenu un rendement quantitatif du produit attendu sous la forme d'une solution à 75% dans l'eau. Sa structure a été confirmée par analyse RMN.
RMN ¹³C (D₂O) : δ 167,6(s), 134,2(d), 134,1(d), 133,8(t), 130,6(s), 129,6(s), 127,3(d), 68,8(t), 67,0(d), 63,2(t), 59,1(t), 58,6(t), 50,8(q), 47,2(q), 16,4(q).

### EXEMPLE 5 : Synthèse du tétrachlorure de N,N-(2-acryloyloxypropane-1,3-diyl)-bis-(N'-i-propyl-N,N,N',N'-tétraméthylbenzène-1,4-diméthanaminium)

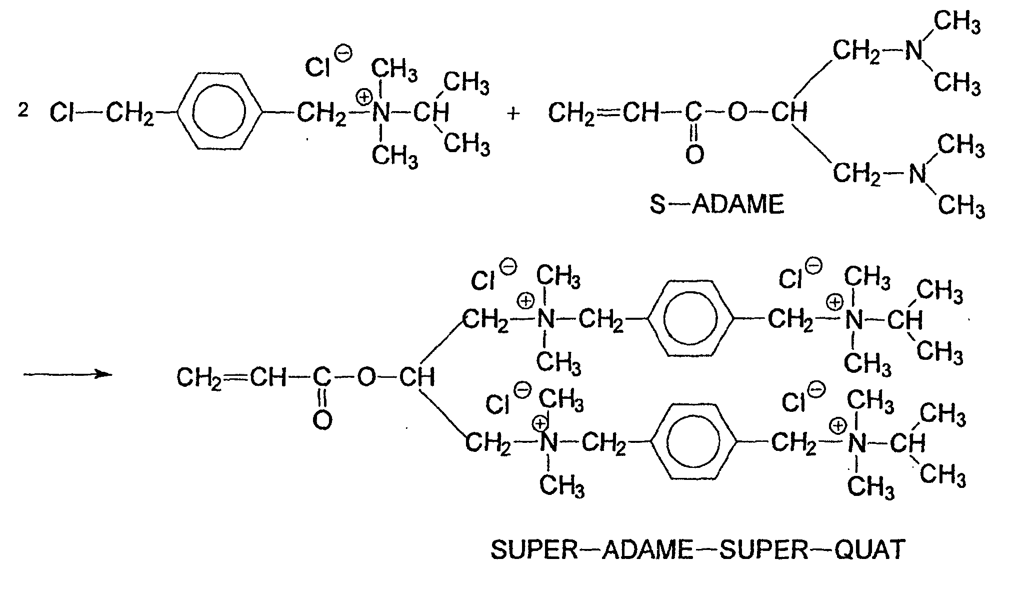

Dans un flacon en verre à large embouchure de 25 ml, équipé d'un capuchon et d'un barreau d'agitation magnétique, on a placé 5,00 g (19,1 mmoles) du composé obtenu à l'Exemple 2, 1,87 g (9,35 mmoles) de S-ADAME, 2,26 g d'eau et 9,03 mg (1000 ppm) d'EMHQ.

Le mélange a ensuite été agité sans chauffage ni refroidissement. La température interne s'est tout d'abord élevée jusqu'à 40-45°C en raison de l'effet exothermique, puis elle a commencé à chuter. Au bout de 30 minutes d'agitation à la température ambiante, un mélange homogène trop visqueux pour être agité s'est formé. On a encore ajouté 0,64 g d'eau pour liquéfier le mélange réactionnel, et après encore 30 minutes d'agitation, une analyse de chlorure ionique a montré que la réaction était complète.

On a obtenu un rendement quantitatif du produit attendu sous la forme d'une solution à 70% dans l'eau.
RMN ¹³C (D₂O) : δ 166,4(s), 136,8(t), 134,3(d), 134,2(d), 130,8(s), 129,0(s), 126,5(d), 70,0(t), 67,0(d), 66,1(t), 64,4(t), 63,8(d), 51,2(q), 50,7(q), 47,3(q), 16,4(q).

## Revendications

1. Composé de formule (Ia) ou (Ib) : 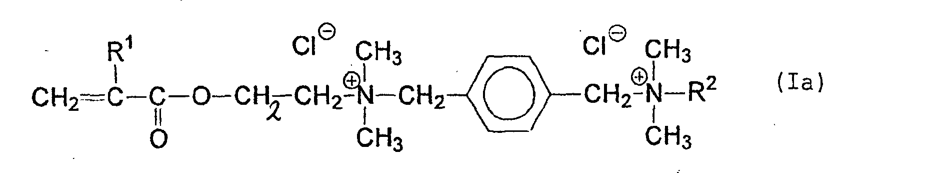 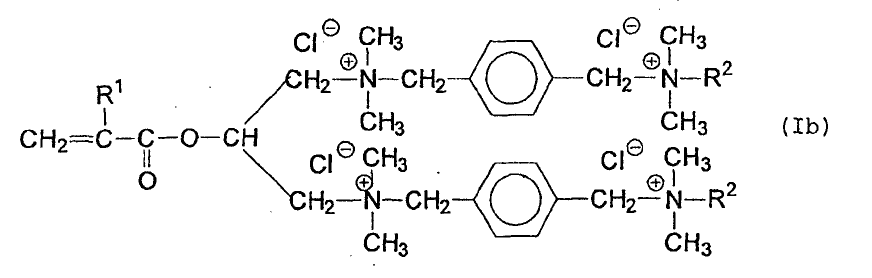 dans lesquelles :
- R¹ représente hydrogène ou méthyle ; et
- R² représente éthyle ou isopropyle.

2. Composé selon la revendication 1, **caractérisé par le fait qu'**il se présente en solution aqueuse, ayant une concentration en composé (Ia) ou (Ib) qui est notamment de 60 à 80% en poids.

3. Procédé de fabrication d'un composé de formule (Ia) ou (Ib) tel que défini à l'une des revendications 1 et 2, **caractérisé par le fait que** :
- dans une première étape, on fait réagir le dichlorure de p-xylylène : 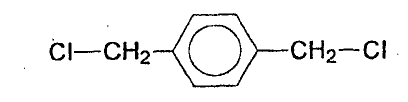 avec un composé de formule (II) : 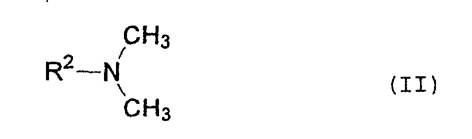 dans laquelle R² est tel que défini à la revendication 1,
pour obtenir un composé de formule (III) : 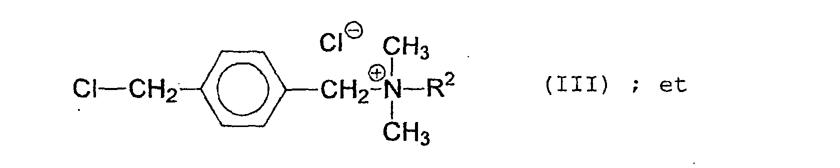
- dans une seconde étape, on fait réagir le composé de formule (III) ainsi obtenu avec un composé de formule (IVa) ou (IVb) : 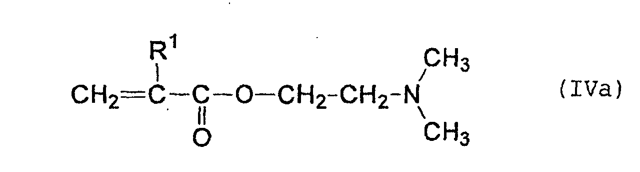 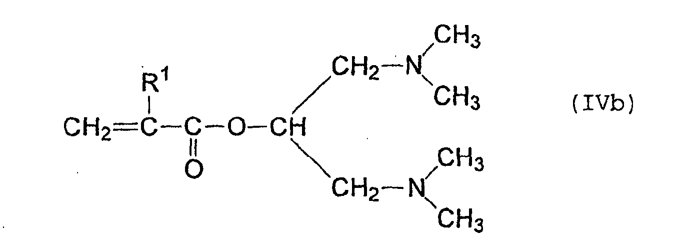
dans lesquelles R¹ est tel que défini à la revendication 1,
conduisant à une solution aqueuse du composé respectivement (Ia) ou (Ib), dont on élimine l'eau le cas échéant.

4. Procédé selon la revendication 3, **caractérisé par le fait qu'**à la première étape, on conduit la réaction dans un milieu solvant.

5. Procédé selon la revendication 4, **caractérisé par le fait que** le solvant est le tétrahydrofuranne.

6. Procédé selon l'une des revendications 3 à 5, **caractérisé par le fait que** l'on conduit la première étape avec un rapport molaire du dichlorure de p-xylylène au composé (II) de 0,8 à 1,2.

7. Procédé selon l'une des revendications 3 à 6, **caractérisé par le fait que** l'on conduit la première étape à une température de 5 à 60°C, pendant un laps de temps de 8 à 144 heures.

8. Procédé selon l'une des revendications 3 à 7, **caractérisé par le fait que** l'on obtient le composé (III) sous la forme de cristaux incolores.

9. Procédé selon l'une des revendications 3 à 8, **caractérisé par le fait que** l'on conduit la deuxième étape avec un rapport molaire du composé (III) au composé (IVa) ou (IVb) compris entre 0,95 et 2,1.

10. Procédé selon l'une des revendications 3 à 9, **caractérisé par le fait que** l'on conduit la deuxième étape à une température de 30 à 40°C, pendant un laps de temps de 1 à 6 heures.

11. Procédé selon l'une des revendications 3 à 10, **caractérisé par le fait que** l'on conduit la réaction de la deuxième étape en présence d'au moins un stabilisant choisi notamment parmi l'éther méthylique de l'hydroquinone, le 3,5-ditert.-butyl-4-hydroxytoluène et l'hydroquinone, et les mélanges de ces stabilisants, à raison de 100 à 2000 ppm par rapport à la charge.

12. Homopolymères ou copolymères comportant des motifs d'au moins un monomère de formule (Ia) ou (Ib) tel que défini à l'une des revendications 1 et 2.

13. A titre de composés intermédiaires dans la préparation des composés des formules (Ia) et (Ib) tels que définis à la revendication 1, les composés de formule (III) tels que définis à la revendication 3.

## Patentansprüche

1. Verbindung der Formel (Ia) oder (Ib): 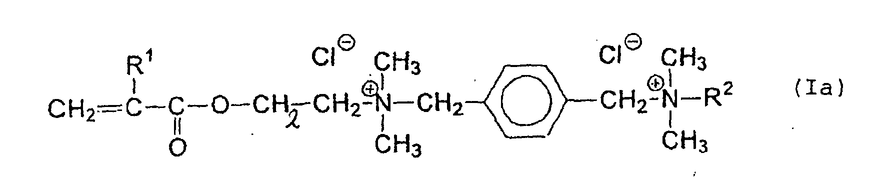 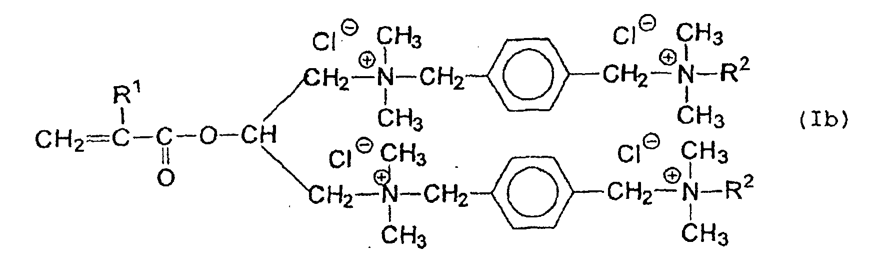 worin:
- R¹ für Wasserstoff oder Methyl steht und
- R² für Ethyl oder Isopropyl steht.

2. Verbindung nach Anspruch 1, **dadurch gekennzeichnet, daß** sie in wäßriger Lösung vorliegt, insbesondere mit einer Konzentration an Verbindung (Ia) oder (Ib) von 60 bis 80 Gew.-%.

3. Verfahren zur Herstellung einer Verbindung der Formel (Ia) oder (Ib) gemäß Anspruch 1 oder 2, **dadurch gekennzeichnet, daß** man:
- in einem ersten Schritt p-Xylylendichlorid: 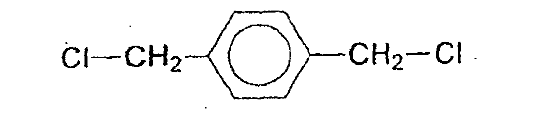 mit einer Verbindung der Formel (II): 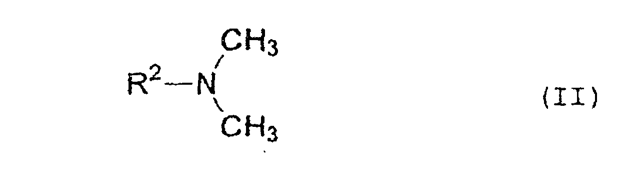 worin R² die in Anspruch 1 angegebene Bedeutung besitzt,
zu einer Verbindung der Formel (III): 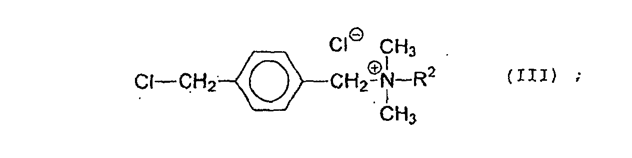 umsetzt und
- in einem zweiten Schritt die so erhaltene Verbindung der Formel (III) mit einer Verbindung der Formel (IVa) oder (IVb): 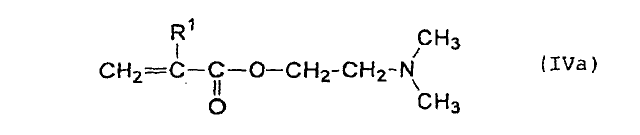 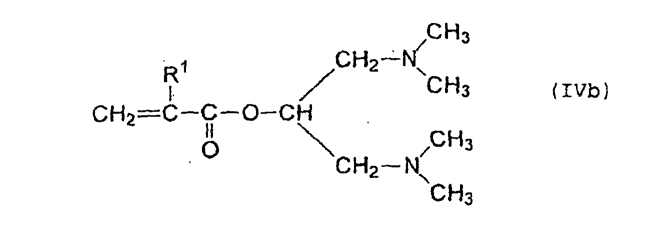
worin R¹ die in Anspruch 1 angegebene Bedeutung besitzt,
umsetzt,
wobei man eine wäßrige Lösung der Verbindung (Ia) bzw. (Ib) erhält, aus der gegebenenfalls das Wasser entfernt wird.

4. Verfahren nach Anspruch 3, **dadurch gekennzeichnet, daß** man im ersten Schritt die Umsetzung in einem Lösungsmittelmedium durchführt.

5. Verfahren nach Anspruch 4, **dadurch gekennzeichnet, daß** man als Lösungsmittel Tetrahydrofuran verwendet.

6. Verfahren nach einem der Ansprüche 3 bis 5, **dadurch gekennzeichnet, daß** man den ersten Schritt mit einem Molverhältnis von p-Xylylendichlorid zu Verbindung (II) von 0,8 bis 1,2 durchführt.

7. Verfahren nach einem der Ansprüche 3 bis 6, **dadurch gekennzeichnet, daß** man den ersten Schritt über einen Zeitraum von 8 bis 144 Stunden bei einer Temperatur von 5 bis 60°C durchführt.

8. Verfahren nach einem der Ansprüche 3 bis 7, **dadurch gekennzeichnet, daß** man die Verbindung der Formel (III) in Form von farblosen Kristallen erhält.

9. Verfahren nach einem der Ansprüche 3 bis 8, **dadurch gekennzeichnet, daß** man den zweiten Schritt mit einem Molverhältnis von Verbindung (III) zu Verbindung (IVa) oder (IVb) zwischen 0,95 und 2,1 durchführt.

10. Verfahren nach einem der Ansprüche 3 bis 9, **dadurch gekennzeichnet, daß** man den zweiten Schritt über einen Zeitraum von 1 bis 6 Stunden bei einer Temperatur von 30 bis 40°C durchführt.

11. Verfahren nach einem der Ansprüche 3 bis 10, **dadurch gekennzeichnet, daß** man die Umsetzung im zweiten Schritt in Gegenwart mindestens eines Stabilisierungsmittels, das insbesondere unter Hydrochinonmethylether, 3,5-Di-tert.-butyl-4-hydroxytoluol und Hydrochinon und Gemischen dieser Stabilisierungsmittel ausgewählt wird, in einem Anteil von 100 bis 2000 ppm, bezogen auf die Charge, durchführt.

12. Homopolymere oder Copolymere mit Einheiten aus mindestens einem Monomer der Formel (Ia) oder (Ib) gemäß Anspruch 1 oder 2.

13. Verbindungen der Formel (III) gemäß Anspruch 3 als Zwischenverbindungen bei der Herstellung der Verbindungen der Formel (Ia) und (Ib) gemäß Anspruch 1.

## Claims

1. Compound of formula (Ia) or (Ib): 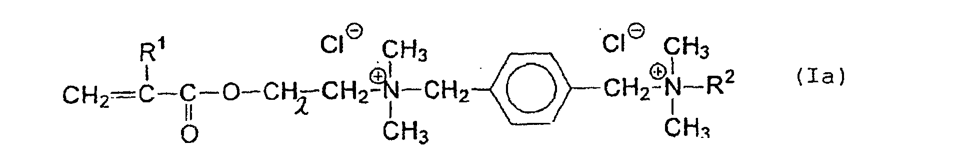 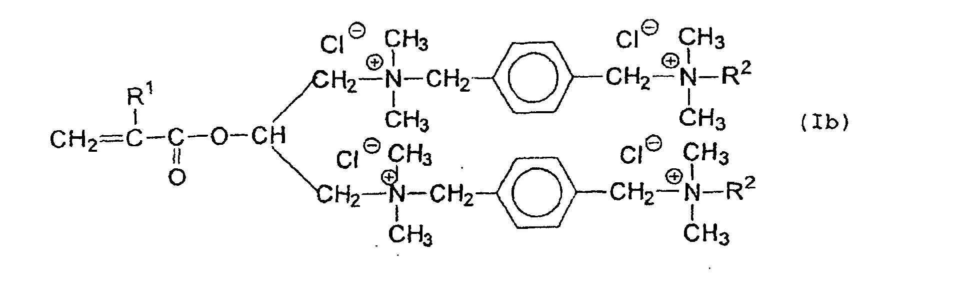 in which:
- R¹ represents hydrogen or methyl; and
- R² represents ethyl or isopropyl.

2. Compound according to Claim 1, **characterized in that** it is provided in aqueous solution, having a concentration of compound (Ia) or (Ib) which is in particular from 60 to 80% by weight.

3. Process for the manufacture of a compound of formula (Ia) or (Ib) as defined in either of Claims 1 and 2, **characterized in that**:
- in a first stage, p-xylylene dichloride: 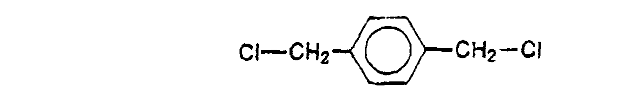 is reacted with a compound of formula (II): 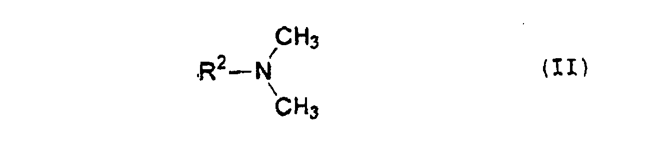 in which R² is as defined in Claim 1,
to produce a compound of formula (III): 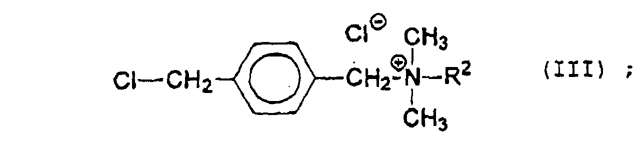 and
- in a second stage, the compound of formula (III) thus obtained is reacted with a compound of formula (IVa) or (IVb): 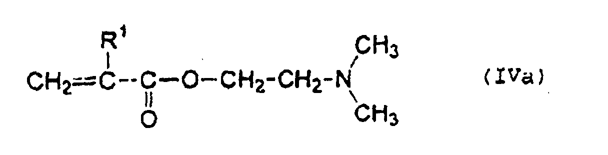 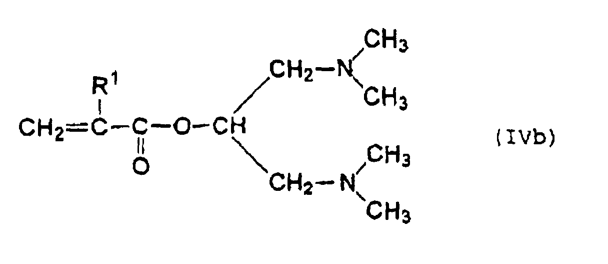
in which R¹ is as defined in Claim 1,
resulting in an aqueous solution of the compound (Ia) or (Ib) respectively, the water of which is removed, if appropriate.

4. Process according to Claim 3, **characterized in that**, in the first stage, the reaction is carried out in a solvent medium.

5. Process according to Claim 4, **characterized in that** the solvent is tetrahydrofuran.

6. Process according to one of Claims 3 to 5, **characterized in that** the first stage is carried out with a molar ratio of the p-xylylene dichloride to the compound (II) of 0.8 to 1.2.

7. Process according to one of Claims 3 to 6, **characterized in that** the first stage is carried out at a temperature of 5 to 60°C for a period of time of 8 to 144 hours.

8. Process according to one of Claims 3 to 7, **characterized in that** the compound (III) is obtained in the form of colourless crystals.

9. Process according to one of Claims 3 to 8, **characterized in that** the second stage is carried out with a molar ratio of the compound (III) to the compound (IVa) or (IVb) of between 0.95 and 2.1.

10. Process according to one of Claims 3 to 9, **characterized in that** the second stage is carried out at a temperature of 30 to 40°C for a period of time of 1 to 6 hours.

11. Process according to one of Claims 3 to 10, **characterized in that** the reaction of the second stage is carried out in the presence of at least one stabilizer chosen in particular from hydroquinone methyl ether, 3,5-di(tert-butyl)-4-hydroxytoluene and hydroquinone, and the mixtures of these stabilizers, in a proportion of 100 to 2 000 ppm with respect to the charge.

12. Homopolymers or copolymers comprising units of at least one monomer of formula (Ia) or (Ib) as defined in either of Claims 1 and 2.

13. The compounds of formula (III) as defined in Claim 3 as intermediate compounds in the preparation of the compounds of formulae (Ia) and (Ib) as defined in Claim 1.
